# EUROPEAN PATENT APPLICATION

(11) **EP 4 006 913 A1**
(43) Date of publication of application: **01.06.2022**
(21) Application number: 21210514.2
(22) Date of filing: 25.11.2021
(51) Int. Cl.: G16H 20/70, A61B 5/16, G06N 3/04

(54) **PERSONALIZED CONTENT PROVIDING METHOD BASED ON PERSONAL MULTIPLE FEATURE INFORMATION AND ANALYSIS APPARATUS**

(30) Priority: 26.11.2020 KR 20200160577
(71) Applicant: NGeneBio Co., Ltd., Guro-gu Seoul 08390 (KR)
(72) Inventor: Yang, Sung Woo, 14563 Gyeonggi-do (KR)
(74) Representative: Ostertag & Partner Patentanwälte mbB

(57) **Abstract**

Disclosed is a method of providing customized content using user multi-feature information, the method including allowing an analysis apparatus to determine a psychological type of a specific individual by inputting genome information of the specific individual to a first learning model, allowing the analysis apparatus to determine a mental health state of the specific individual by inputting the genome information, clinical information, and lifestyle habit information of the specific individual to a second learning model, and allowing the analysis apparatus to determine customized digital content for the specific individual matching the psychological type and the mental health state with reference to a content database.

## Description

### CROSS-REFERENCE TO RELATED APPLICATION

This application claims the benefit under 35 U.S.C. § 119(a) of Korean Patent Application No. 10-2020-0160577, filed on November 26, 2020, in the Korean Intellectual Property Office, the entire disclosure of which is incorporated herein by reference for all purposes.

### BACKGROUND

### 1. Field of the Invention

The following description relates to a technique for providing customized content according to an individual's psychological state and mental health state using user multi-feature information.

### 2. Discussion of Related Art

Customized services are provided in various fields. Typically, intelligence technology (IT) companies provide services that expose advertisements, products, and content tailored to individuals using collected personal information. However, most of the customized services are limited to providing content based on individuals' feedback, activity information, and preference through the Internet.

### SUMMARY OF THE INVENTION

The following description is directed to providing a technique for estimating an individual's psychological type or mental health state on the basis of bio-information of the individual such as the individual's genome information and clinical information and providing customized content based on the estimated result. Furthermore, the following description is directed to providing a technique for providing customized content based on multi-information for an individual by additionally using the individual's lifestyle habit information, etc.

In one general aspect, there is provided a method of providing customized content using user multi-feature information, the method including allowing an analysis apparatus to determine a psychological type of a specific individual by inputting genome information of the specific individual to a first learning model, allowing the analysis apparatus to determine a mental health state of the specific individual by inputting the genome information, clinical information, and lifestyle habit information of the specific individual to a second learning model, allowing the analysis apparatus to determine customized digital content for the specific individual matching the psychological type and the mental health state with reference to a content database, and allowing the analysis apparatus to provide the customized digital content.

In another general aspect, there is provided an apparatus for analyzing customized content using user multi-feature information, the apparatus including an input device configured to receive genome information, clinical information, and lifestyle habit information for a specific individual, a storage device configured to store a first learning model that receives the genome information and outputs a psychological type of an analysis target and a second learning model that receives the genome information, the clinical information, and the lifestyle habit information and outputs a mental health state of the analysis target, and a computing device configured to determine a psychological type of the specific individual by inputting the genome information of the specific individual to the first model learning model, configured to determine the mental health state of the specific individual by inputting the genome information, the clinical information, and the lifestyle habit information of the specific individual to the second learning model, and configured to determine customized digital content for the specific individual matching the psychological type and the mental health state with reference to a content database.

### BRIEF DESCRIPTION OF THE DRAWINGS

FIG. 1 is an exemplary customized content provision system;
FIG. 2 is another exemplary customized content provision system;
FIG. 3 is an example operation of an analysis apparatus;
FIG. 4 is an example of a psychological-type estimation process through a first learning model;
FIG. 5 is an example of a health state estimation process through a second learning model;
FIG. 6 is an example of a customized content provision process;
FIG. 7 is an example of feature information, which is a criterion for determining customized content and
FIG. 8 is an example of an analysis apparatus.

Throughout the drawings and the detailed description, unless otherwise described, the same drawing reference numerals will be understood to refer to the same elements, features, and structures. The drawings may not be to scale, and the relative size, proportions, and depiction of elements in the drawings may be exaggerated for clarity, illustration, and convenience

### DETAILED DESCRIPTION OF EXEMPLARY EMBODIMENTS

As the following description may be variously modified and have several example embodiments, specific embodiments will be shown in the accompanying drawings and described in detail below. It should be understood, however, that there is no intent to limit the following description to the particular forms disclosed, but on the contrary, the following description is to cover all modifications, equivalents, and alternatives falling within the spirit and scope of the invention.

In addition, the terms such as "first," "second," "A," and "B" may be used to describe various elements, but these elements are not limited by these terms. These terms are used to only distinguish one element from another element. For example, a first element may be called a second element, and a second element may also be called a first element without departing from the scope of the following description. The term "and/or" means any one or a combination of a plurality of related items.

It should be understood that singular forms are intended to include plural forms unless the context clearly indicates otherwise, and it should be further understood that the terms "comprise," "include," or "have" as used herein specify the presence of stated features, numerals, steps, operations, elements, components, or a combination thereof but do not preclude the presence or addition of one or more other features, numerals, steps, operations, elements, components, or combinations thereof.

Prior to a detailed description of the drawings, it should be clarified that division of components in the present specification is performed merely based on main functions performed by the respective components. That is, two or more components which will be described later may be integrated into a single component or, alternatively, a single component may be divided into two or more components depending on subdivided functions. Further, it is apparent that each of the components, which will be described later, may additionally perform some or all of the functions performed by other components in addition to main functions performed thereby, and some of the main functions performed by the respective components may be shared with other components and may be performed.

In addition, the respective steps of the above method may be performed in a sequence different from a described sequence unless the context clearly defines a specific sequence. That is, the respective steps may be performed in the same order as described, substantially simultaneously, or in reverse order.

A host is an object that microorganisms inhabit. Here, the host is assumed to be a human.

A sample refers to a specimen extracted from the host. The sample is a specimen for determining host genome information or disease information, such as blood or tissue. Also, the sample may be a specimen capable of identifying intestinal microorganisms, such as feces.

Genome information refers to genome data of a specific individual or entity. The genome information may be acquired using various techniques. The entity basically includes humans, animals, plants, microorganisms, and the like. For example, the genome information may include nucleotide sequences, gene expression data, genetic variation with standard genome data, DNA methylation, and the like which are obtained from deoxyribonucleic acid (DNA), ribonucleic acid (RNA), or protein from cells, tissues, etc. Typically, the genome information may be gene information obtained using next-generation sequencing (NGS).

The genome information may include epigenome information. The epigenome information refers to factors that affect gene expression and includes information related to DNA methylation, histone modification, microRNA, and the like.

A microbiome is defined as any microbial communities such as bacteria, viruses, and fungi that inhabit and coexist in a human body at the individual level and genetic information of these microbial communities. In general, the human microbiome includes information about microorganisms that inhabit the human intestine, oral cavity, skin, and the like. The human microbiome includes information such as the whole metagenome along with the 16s rRNA of the microorganisms. The amount of 16s rRNA can be determined by performing metagenome analysis on various samples from humans. Metagenome analysis may be performed using various bioinformatics tools related to the metagenome analysis. The purpose of the analysis is to determine the function and distribution of microorganisms derived from a user's body, usually by measuring the amount of microorganisms. The human microbiome includes operational taxonomic unit (OTU) information obtained from raw information for 16s rRNA analysis. Furthermore, whole-genome sequencing is a method of decoding the entire nucleotide sequence of a sample, and the result of the analysis includes both of human genetic information and microbial genetic information. Therefore, a researcher can check genetic information of a microbial community by extracting only the microbial genetic information (the filtering of the human genetic information).

An individual's genome information may be used to include a host's genome information and microbiome information.

Clinical information may include various items such as biometric information, medical treatment information, and prescription information about an individual (patient). For example, clinical information may include an individual's body information (age, gender, race, height, weight, etc.), examination information (blood test results, X-ray images, CT images, MRI images, electrocardiogram tests, genomic analysis results, etc.), diagnosis results (health states, disease information, etc.). Meanwhile, the clinical information may also include medical treatment information and diagnosis results on a specific individual's family. That is, the clinical information may include medical treatment information, diagnosis results, etc. of not only a specific individual but also genetically related others.

Meanwhile, the clinical information may include questionnaire information and survey information for measuring personal health.

The survey information may include at least a plurality of items from the item group consisting of stress resilience, inflammatory response, neurological function, boost energy-level, blood glucose response to meals, body mass composition, sleep, nutrient absorption, physical fatigue, and food allergy. The survey information may include information about an individual's stress and emotional state.

Lifestyle habit information refers to information related to lifestyle habits. For example, the lifestyle habit information may include (i) physical activity information, (ii) food intake information, (iii) exercise information, and the like. The physical activity information may include sleep time, wake up time, work time, rest time, and the like. The food intake information may include a daily calorie intake, the amount of intake nutrients, and the like. The exercise information may include information on whether to exercise, the intensity of exercise, and the like. The lifestyle habit information may be collected through a smart device, an Internet of Things (IoT) device, a wearable device, etc.

The lifestyle habit information may include information such as sleep amount, sleep time, sleep pattern, exercise amount, exercise time, walking time, walking distance, and the like. Furthermore, the lifestyle habit information may include personal sound information. The sound information may include voice, snoring, etc.

Psychological type information refers to information obtained by classifying an individual's inclination or psychology. For example, the psychological type information may be information (16 classifications) such as the Myers-Briggs type indicator (MBTI). Psychological type information may be used as information obtained by classifying identification information about an individual.

Hereinafter, information that can characterize a specific individual in relation to health and psychology is referred to as user multi-feature information (or is abbreviated as multi-feature information). Accordingly, the user multi-feature information may include genome information, clinical information, lifestyle habit information, psychological type, and the like about an individual.

Meanwhile, the following description may estimate an individual's health state (mental health state) or psychological type using a learning model. The learning model refers to a machine learning model. The learning model includes various types of models. For example, the learning model may be a decision tree, a random forest, a K-nearest neighbor (KNN), a naive Bayes, a support vector machine (SVM), an artificial neural network, and the like. As for artificial neural networks, deep neural networks (DNNs) are attracting attention recently. DNNs include a convolutional neural network (CNN), a recurrent neural network (RNN), a restricted Boltzmann machine (RBM), a deep belief network (DBN), a generative adversarial network (GAN), relation networks (RLs), and the like.

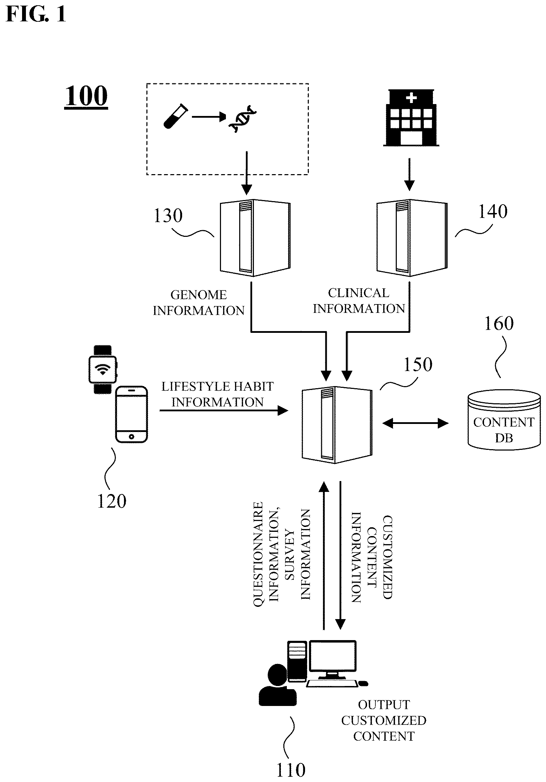

FIG. 1 is an exemplary customized content provision system 100.

An analysis server 150 determines customized content for a specific individual. The analysis server 150 needs the above-described user multi-feature information.

A user terminal 110 receives and transmits questionnaire information or survey information for a specific individual. The questionnaire information and the survey information may include items that a service provider wants to check in advance.

A wearable device 120 transmits lifestyle habit information of a specific individual. Various devices such as a smart device and an IoT device may collect and transmit lifestyle habit information. Meanwhile, the user terminal 110 may collect information collected by peripheral devices and transmit the lifestyle habit information.

A genome information server 130 transmits genome information of a specific individual. The genome information server 130 may analyze a sample of the specific individual and generate the genome information. The description of a process of collecting samples and analyzing genome will be omitted. The genome information may be composite information including a host's whole genome and whole metagenome or sequence information of a specific target and a specific marker (16s rRNA Sequencing). Furthermore, the genome information may include information on an epigenome, a transcriptome, and a proteome.

A medical information server 130 transmits clinical information of a specific individual. A medical institution may transmit clinical information including a specific individual's medical treatment result, test result, disease information, and the like. The clinical information may include medical treatment information on a specific individual's family.

In some cases, the medical information server 130 other than the genome information server 130 may also transmit genome information of a specific individual. Also, the medical information server 130 may transmit questionnaire information or survey information collected during a medical treatment process.

The analysis server 150 receives survey information, lifestyle habit information, genome information, clinical information, etc. That is, the analysis server 150 receives user multi-feature information.

The analysis server 150 may determine the genotype of the specific individual on the basis of the genome information. The genotype refers to information that can identify or characterize the specific individual on the basis of the genome information (host genome information and microbiome). The genotype may be determined based on at least one genome information item including whether specific genes are expressed, the amount of expression of specific genes, expression patterns of a plurality of genes, epigenomic features, the composition of an intestinal microbial community, and the types of intestinal microorganisms.

The analysis server 150 estimates the psychological type and health state of the specific individual to be analyzed by inputting the received information to a learning model trained in advance. Various models such as a statistical analysis model and a neural network may be used as the learning model. The health state includes a mental health state.

The learning model may be a model for extracting feature information from the user multi-feature information. Alternatively, the learning model may generate a deviation value for an individual's health state by comparing the input information to a reference value on the basis of the user multi-feature information.

Based on an output value of the learning model, the analysis server 150 may determine customized content related to the output value with reference to a content database 160.

The analysis server 150 may acquire information specifying a specific individual from the user multi-feature information. The information is referred to as analysis target feature information or feature information. The analysis target feature information may include at least one of a value output by the learning model and the genotype of a specific individual. The analysis target feature information may be information obtained by processing at least one of a value output by the learning model and the genotype of a specific individual.

Based on the analysis target feature information, the analysis server 150 may determine customized content information related to a specific individual from the content DB 160. The customized content corresponds to content that can promote or alleviate a health state according to the psychological type and health state of a specific target.

The analysis server 150 delivers the customized content information to the user terminal 110. The user terminal 110 outputs the customized content. As will be described below, the customized content includes visual content. The customized content may include a color, a background color, an object design, a font type, and the like of an object output to a screen. For example, the user terminal 110 may change and output a background color or a background image according to the customized content information.

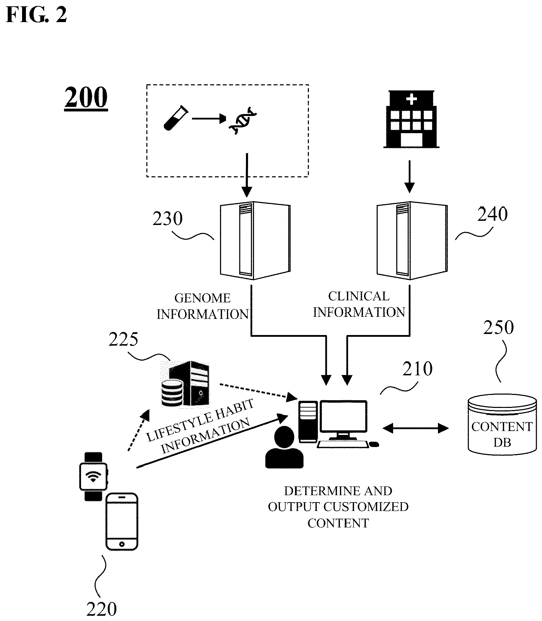

FIG. 2 is another exemplary customized content provision system 200. FIG. 2 is an embodiment in which a personal terminal (a computer device, a personal computer (PC), a notebook, a smart device, etc.) determines and provides customized content.

A user terminal 210 receives questionnaire information or survey information from a specific individual. The questionnaire information and the survey information may include items that a service provider wants to check in advance.

A wearable device 220 transmits lifestyle habit information of a specific individual. Various devices such as a smart device and an IoT device may collect and transmit lifestyle habit information. The wearable device 220 may transmit the lifestyle habit information to a separate service server 225 that collects the lifestyle habit information. In this case, the user terminal 110 may receive the lifestyle habit information from the service server 225.

A genome information server 230 may analyze a sample of the specific individual and generate genome information. The genome information server 230 transmits genome information of a specific individual.

A medical information server 230 transmits clinical information of a specific individual. A medical institution may transmit clinical information including a specific individual's medical treatment result, test result, disease information, and the like. The clinical information may include medical treatment information on a specific individual's family.

In some cases, the medical information server 230 other than the genome information server 230 may also transmit genome information of a specific individual. Also, the medical information server 230 may transmit questionnaire information or survey information collected during a medical treatment process.

The user terminal 210 receives survey information, lifestyle habit information, genome information, clinical information, etc. That is, the user terminal 210 receives user multi-feature information.

The user terminal 210 may determine the genotype of the specific individual on the basis of the genome information. The genotype refers to information that can identify or characterize the specific individual on the basis of the genome information (host genome information and microbiome). The genotype may be determined based on at least one genome information item including whether specific genes are expressed, the amount of expression of specific genes, expression patterns of a plurality of genes, epigenomic features, the composition of an intestinal microbial community, and the types of intestinal microorganisms.

The user terminal 210 estimates the psychological type and health state of the specific individual to be analyzed by inputting the received information to a learning model trained in advance. Various models such as a statistical analysis model and a neural network may be used as the learning model. The health state includes a mental health state.

The learning model may be a model for extracting feature information from the user multi-feature information. Alternatively, the learning model may generate a deviation value for an individual's health state by comparing the input information to a reference value on the basis of the user multi-feature information.

Based on an output value of the learning model, the user terminal 210 may determine customized content related to the output value with reference to a content database 250.

Based on the analysis target feature information, the user terminal 210 may determine customized content information related to a specific individual from the content DB 250. The customized content corresponds to content that can promote or alleviate a health state according to the psychological type and health state of a specific target.

The user terminal 210 outputs the customized content. For example, the user terminal 110 may change and output a background color or a background image according to the customized content information.

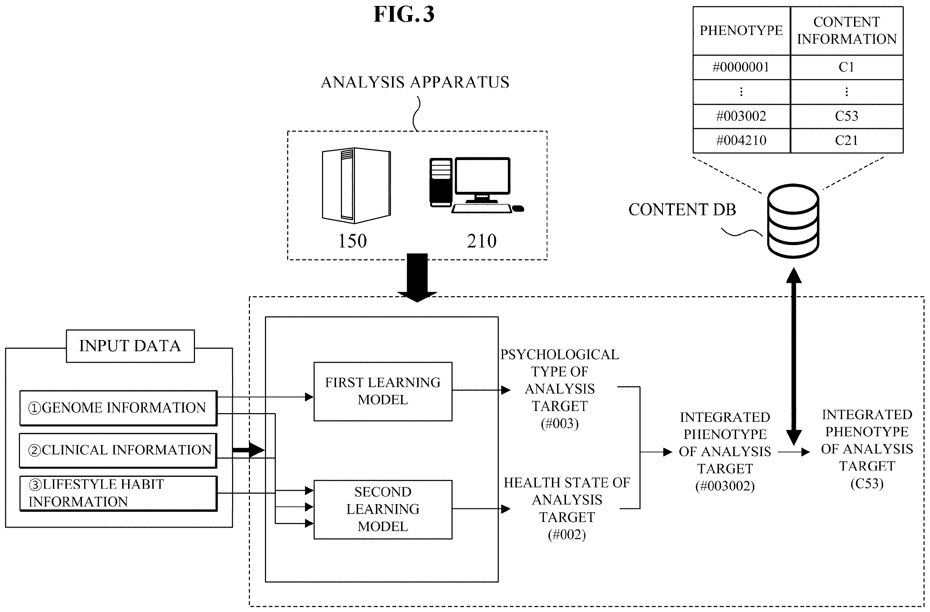

FIG. 3 is an example operation of an analysis apparatus. The analysis apparatus is an apparatus corresponding to the analysis server 150 of FIG. 1 or the personal terminal 210 of FIG. 2.

The analysis apparatus receives input data. The input data includes ① genome information, ② clinical information, and ③ lifestyle habit information.

The analysis apparatus estimates the psychological type and health state of an analysis target using a learning model. In this case, the analysis apparatus may estimate a psychological type and a health state using a first learning model and a second learning model.

The first learning model may receive input data and output the psychological type of the analysis target. The second learning model may receive input data and output the health state information of the analysis target. The health state includes mental health information.

The first learning model may receive genome information and output the psychological type (#003) of the analysis target. #003 denotes identification information indicating a specific type among psychological types.

The second learning model may receive genome information, clinical information, and lifestyle habit information and output the health state (#002) of the analysis target. #002 denotes identification information indicating a specific health state among health states.

The analysis apparatus may generate integrated information by merging the psychological type (#003) and the health state (#002) of the analysis target. The integrated information about the analysis target is referred to as an integrated phenotype. FIG. 3 is an example of determining customized content for an analysis target on the basis of an integrated phenotype (#003002).

The analysis apparatus searches for matching content information with reference to a content DB on the basis of the integrated phenotype of the analysis target. Content information for #003002 is assumed to be C53. The analysis apparatus determines customized content information C53 of the analysis target.

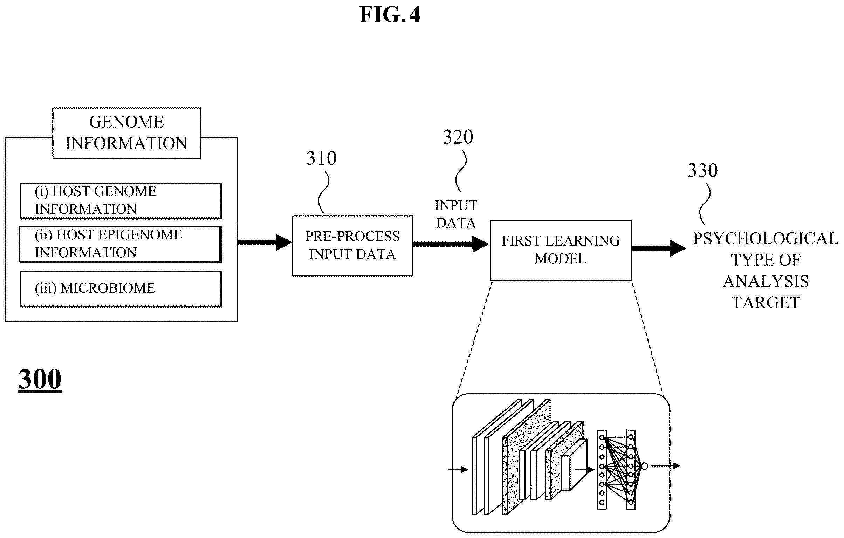

FIG. 4 is an example of a psychological-type estimation process 300 through a first learning model. The first learning model estimates the psychological type of an analysis target by using genome information.

The genome information includes host genome information, host epigenome information, and a microbiome. An analysis apparatus may uniformly pre-process genome information (310).

The analysis apparatus may choose a gene associated with an individual's psychological type among the genome information. The gene associated with the psychological type may be determined using the known literature information or research results. The analysis apparatus may perform subsequent processes on the chosen gene.

The analysis apparatus may normalize different pieces of genome information. Also, the analysis apparatus may uniformly integrate different pieces of genome information. The pre-processing of the input data may vary depending on the structure and type of the learning model.

For example, (i) when the first learning model receives a one-dimensional (1D) array (vector), the analysis apparatus may pre-process a plurality of pieces of genome information into a 1D vector sequence. (ii) When the first learning model receives a two-dimensional (2D) matrix such as a CNN, the analysis apparatus may pre-process a plurality of pieces of genome information in the form of a 2D matrix. In this case, depending on the type of genome information, the analysis apparatus may generate a plurality of matrices or integrate the genome information to generate one matrix. This varies depending on an input layer structure (one input layer or a plurality of input layers) of the first learning model.

The analysis apparatus inputs the pre-processed input data to the first learning model (320). In this case, the first learning model is assumed to be a model that has been trained in advance. The first learning model analyzes the input data and outputs information on the psychological type of the analysis target (330).

FIG. 4 shows the neural network model as an example of the first learning model. However, the first learning model may be one of the various learning models. For example, the first learning model may be one of the models such as KNN, SVM, decision tree, random forest, and deep learning model. The first learning model outputs information on psychological types, and when the psychological types are classified into many (16) groups like MBTI, the first learning model should be able to determine one of the various types. For example, as the first learning model, one individual model may be provided for each psychological type classification. Representatively, if described using a random forest model, the first learning model may include a first model for a first psychological type, a second model for a second psychological type, ..., and an i^{th} model for an i^{th} psychological type. Here, i is the number of psychological types. The individual models receive the same input data and output respective output values. The analysis apparatus may determine the psychological type of the analysis target on the basis of a model expected to have the highest accuracy among the values output by the individual models. For example, when an individual model outputs a value between 0 and 1 and an output value of a kth model (k-type) is highest (as 0.91), the analysis apparatus may determine that the psychological type of the analysis target is the k-type.

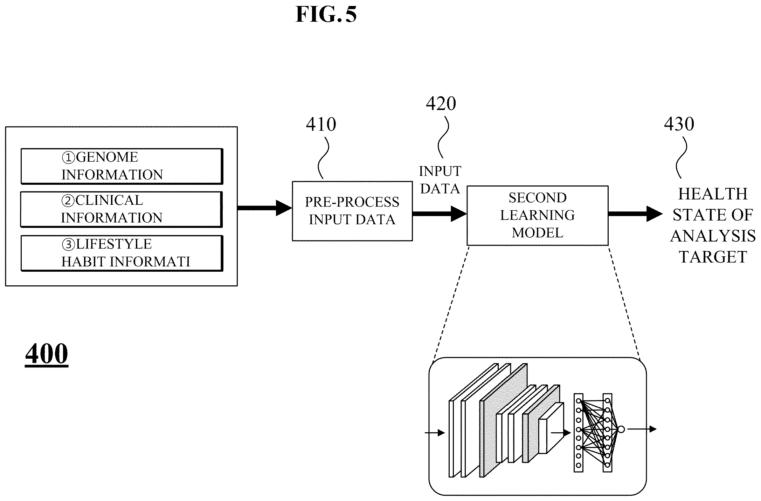

FIG. 5 is an example of a health state estimation process 400 through a second learning model. The second learning model estimates the health state of the analysis target by using input data. Hereinafter, the health state will be described with a focus on a psychological health state. The psychological health state may be at least one of apathy, depression, stress, chronic fatigue, and anxiety.

The input data may include genome information, clinical information, and lifestyle habit information. The genome information includes host genome information, host epigenome information, and microbiome. An analysis apparatus may uniformly pre-process input data (410). The analysis apparatus may normalize different types of information. Each of the genome information, the clinical information, and the lifestyle habit information includes a plurality of items. Accordingly, the analysis apparatus may integrate information of the plurality of items to form all of the genome information, all of the clinical information, and all of the lifestyle habit information. Furthermore, the analysis apparatus may configure the genome information, the clinical information, and the lifestyle habit information into single integrated information.

The pre-processing of the input data may vary depending on the structure and type of the learning model.

For example, (i) when the second learning model receives one-dimensional (1D) array (vector), the analysis apparatus may pre-process a plurality of pieces of input data into a 1D vector sequence. (ii) When the second learning model receives a two-dimensional (2D) matrix such as a CNN, the analysis apparatus may pre-process a plurality of pieces of input data in the form of a 2D matrix. In this case, depending on the type of input data, the analysis apparatus may generate a plurality of matrices or integrate the input data to generate one matrix. This varies depending on an input layer structure (one input layer or a plurality of input layers) of the second learning model.

The analysis apparatus inputs the pre-processed input data to the second learning model (420). In this case, the second learning model is assumed to be a model that has been trained in advance. The second learning model analyzes the input data and outputs information on the psychological health state of the analysis target (430).

FIG. 4 shows the neural network model as an example of the second learning model. However, the second learning model may be one of the various learning models. For example, the second learning model may be one of the models such as KNN, SVM, decision tree, random forest, and deep learning model. The second learning model outputs information on at least one health state among a plurality of mental health states, and the second learning model should be able to determine one health state among various health states.

For example, as the second learning model, one individual model may be provided for each mental health state. Representatively, if described using a random forest model, the second learning model may include a first model for a first mental health state, a second model for a second mental health state, ..., and a j^{th} model for a j^{th} mental health state. Here, j is the number of mental health states. The individual models receive the same input data and output respective output values. The analysis apparatus may determine the mental health state of the analysis target on the basis of a model expected to have the highest accuracy among the values output by the individual models. For example, when an individual model outputs a value between 0 and 1 and an output value of an m^{th} model (m-health state) is highest (as 0.92), the analysis apparatus may determine that the mental health state of the analysis target is the m-health state.

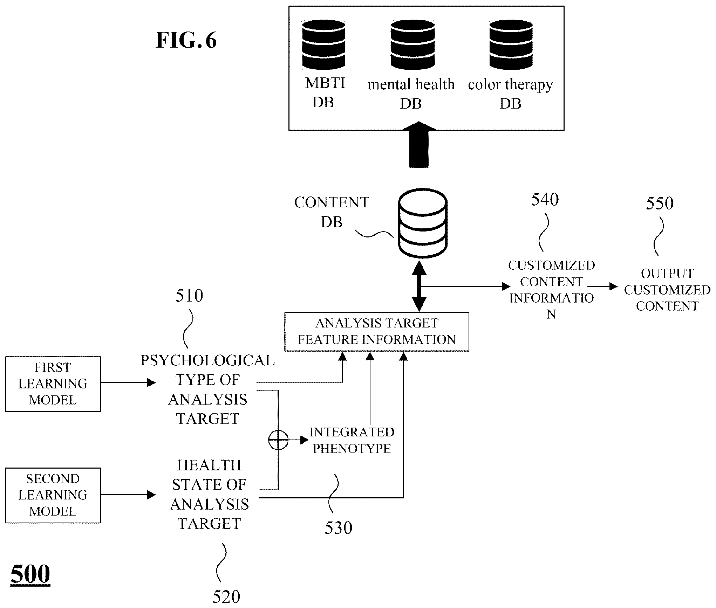

FIG. 6 is an example of a customized content provision process 500.

As described above, an analysis apparatus estimates the psychological type of an analysis target as a first learning model using input data (510). Also, the analysis apparatus estimates the mental health state of the analysis target as a second learning model using input data (520). The analysis apparatus may generate an integrated phenotype by combining a psychological type and health state information (S530).

The analysis apparatus uses analysis target feature information as information specifying a specific individual which is to be analyzed. The analysis target feature information may be configured as at least one of the psychological type, mental health state, and integrated phenotype of the analysis target.

Based on the derived analysis target feature information, the analysis apparatus may determine information on customized content matching the corresponding information with reference to a content DB (540). The content DB may include information regarding various items. The content DB may include a psychological type (e.g., MBTI DB), a mental health DB, a color therapy DB, and the like. The MBTI DB may hold customized content information according to psychological types. The mental health DB may hold customized content information according to mental health states. The color therapy DB may hold information on content (such as color values) that is used to induce an individual's psychological or mental health to a normal state according to a psychological state or a mental health state.

The customized content may be visual content that induces a psychological or mental state of a specific individual to a normal state or a relaxed state. For example, the customized content may include at least one of a background image, a background color, an object color, a text font, and an object design which are output to a display.

The analysis apparatus may output the determined customized content (550). Alternatively, the analysis apparatus may transmit the determined customized content information to a personal terminal so that the personal terminal outputs the customized content.

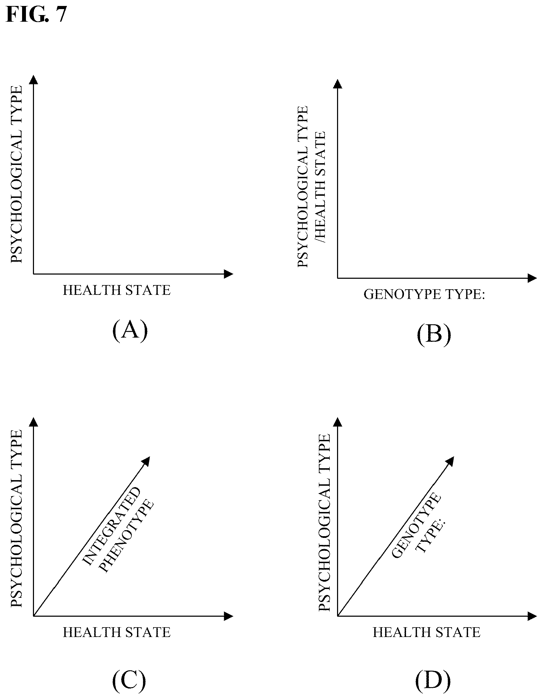

FIG. 7 is an example of feature information, which is a criterion for determining customized content. A criterion for finding customized content in a content DB is referred to as analysis target feature information or feature information. Various values may be used as the feature information.

FIG. 7A is an example in which a health state and a psychological type are used. The feature information may have the form of a 2D matrix. One axis indicates the health state, and the other axis indicates the psychological type. Accordingly, the feature information may be expressed as (x, y).

FIG. 7B is an example in which a genotype type and a health state or psychological type are used. The genotype is the same as described above. The feature information may have a 2D matrix form. One axis may indicate a genotype type, and the other axis may be one of a health state and a psychological type. Alternatively, one axis may indicate a genotype type, and the other axis may indicate an integrated phenotype. The feature information may be expressed as (x, y).

FIG. 7C is an example in which a health state, a psychological type, and an integrated phenotype are used. The feature information may have the form of a three-dimensional (3D) matrix. One axis indicates the health state, another axis indicates the psychological type, and the other axis indicates the integrated phenotype. Accordingly, the feature information may be expressed as (x, y, z).

FIG. 7D is an example in which a health state, a psychological type, and a genotype type are used. The feature information may have the form of a three-dimensional (3D) matrix. One axis indicates the health state, another axis indicates the psychological type, and the other axis indicates the genotype type. Accordingly, the feature information may be expressed as (x, y, z).

The analysis apparatus may search the content DB for corresponding customized content on the basis of the feature information as shown in FIG. 7.

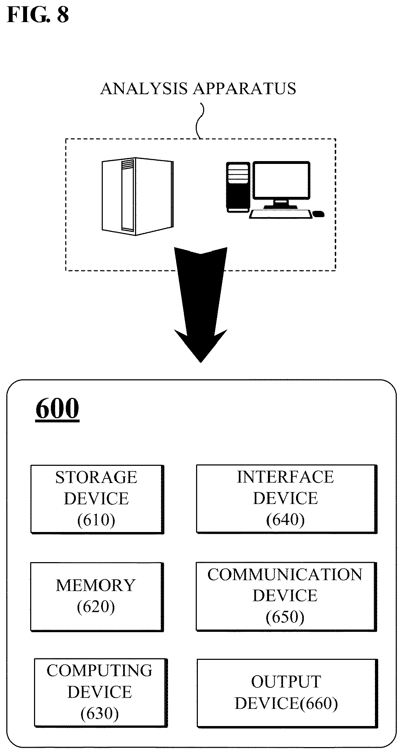

FIG. 8 is an example of an analysis apparatus 600. The analysis apparatus 600 of FIG. 8 is an apparatus corresponding to the analysis server 150 of FIG. 1 or the personal terminal 210 of FIG. 2.

The analysis apparatus 600 is an apparatus for determining customized content for a corresponding individual using user multi-feature information.

The analysis apparatus 600 may be physically implemented in various forms. For example, the analysis apparatus 600 may have the form of a computer device such as a PC, a server on a network, a chipset dedicated to data processing, and the like. The computer device may include a mobile device such as a smart device.

The analysis apparatus 600 may include a storage device 610, a memory 620, a computing device 630, an interface device 640, a communication device 650, and an output device 660.

The storage device 610 may store received input data. The storage device 610 may store the user multi-feature information. The storage device 610 may store genome information, clinical information, and lifestyle habit information for a specific individual. The storage device 610 may store input data that is uniformly pre-processed.

The storage device 610 may store a first learning model that receives genome information and outputs the psychological type of the analysis target and a second learning model that receives genome information, clinical information, and lifestyle habit information and outputs the mental health state of the analysis target.

The storage device 610 may store a content DB.

The storage device 610 may store customized content information or customized content determined for a specific individual.

The memory 620 may store data and information generated while the analysis apparatus 600 analyzes the customized content.

The interface device 640 is an apparatus for receiving some commands and data from the outside. The interface device 640 may receive genome information, clinical information, and lifestyle habit information for a specific individual from an external storage device or an input device physically connected to the interface device 640. The interface device 640 may receive a command for analyzing the user multi-feature information.

The communication device 650 refers to an element for receiving and transmitting some information over a wired or wireless network. The communication device 650 may receive genome information, clinical information, and lifestyle habit information for a specific individual from an external object. The communication device 650 may receive a command for analyzing the user multi-feature information. The communication device 650 may transmit customized content information, which is an analysis result, to an external object.

The communication device 650 may receive relevant content information in communication with a content DB over a network.

The communication device 650 or the interface device 640 is a device for receiving some commands and data from the outside. The communication device 650 or the interface device 640 may be referred to as an input device.

The output device 660 is a device for outputting some information. The output device 660 may output an interface, an analysis result, etc., that is necessary for a data processing process. Also, the output device 660 may output the determined customized content to a screen.

The computing device 630 may analyze input data using a learning model and a program stored in the storage device 610 and may determine customized content.

The computing device 630 may uniformly pre-process input data as described above.

The computing device 630 may determine the psychological type of the specific individual by inputting the genome information of the specific individual to the first learning model.

The computing device 630 may determine the mental health state of the specific individual by inputting the genome information, clinical information, and lifestyle habit information of the specific individual to the second learning model.

The computing device 630 may analyze the genome information and determine the genotype type of the specific individual.

The computing device 630 may generate feature information using a psychological type, a mental health state, an integrated phenotype, and a genotype type. The type of the feature information is as described with reference to FIG. 7.

The computing device 630 may determine customized content information for a specific individual with reference to a content DB on the basis of the feature information.

The computing device 630 may be a device, such as a processor, an application processor (AP), and a chip with an embedded program, for processing data and processing some computations.

The above description provides digital content that can promote or alleviate an individual's mental health state according to the individual's psychological state and health.

Also, the above-described customized content determination and provision methods may be implemented using a program (or application) including an executable algorithm that may be executed by a computer. The program may be stored and provided in a transitory or non-transitory computer-readable medium.

The non-transitory computer-readable medium refers to a medium that semi-permanently stores data and is readable by a device rather than a medium that temporarily stores data such as a register, a cache, and a memory. Specifically, the above-described various applications or programs may be stored and provided in a non-transitory computer-readable medium such as a compact disc (CD), a digital versatile disc (DVD), a hard disk, a Blu-ray disc, a Universal Serial Bus (USB), a memory card, a read-only memory (ROM), a programmable read-only memory (PROM), an erasable PROM (EPROM), an electrically EPROM (EEPROM), or a flash memory.

A transitory computer-readable medium refers to various random-access memories (RAMs) such as a static RAM (SRAM), a dynamic RAM (DRAM), a synchronous DRAM (SDRAM), a double data rate SDRAM (DDR SDRAM), an enhanced SDRAM (ESDRAM), a SyncLink DRAM (SLDRAM), and a Direct Rambus RAM (DRRAM).

While this disclosure includes specific examples, it will be apparent after an understanding of the disclosure of this application that various changes in form and details may be made in these examples without departing from the spirit and scope of the claims and their equivalents. The examples described herein are to be considered in a descriptive sense only, and not for purposes of limitation. Descriptions of features or aspects in each example are to be considered as being applicable to similar features or aspects in other examples. Suitable results may be achieved if the described techniques are performed in a different order, and/or if components in a described system, architecture, device, or circuit are combined in a different manner, and/or replaced or supplemented by other components or their equivalents. Therefore, the scope of the disclosure is defined not by the detailed description, but by the claims and their equivalents, and all variations within the scope of the claims and their equivalents are to be construed as being included in the disclosure.

## Claims

1. A method of providing customized content using user multi-feature information, the method comprising:
allowing an analysis apparatus to determine a psychological type of a specific individual by inputting genome information of the specific individual to a first learning model;
allowing the analysis apparatus to determine a mental health state of the specific individual by inputting the genome information, clinical information, and lifestyle habit information of the specific individual to a second learning model;
allowing the analysis apparatus to determine customized digital content for the specific individual matching the psychological type and the mental health state with reference to a content database; and
allowing the analysis apparatus to provide the customized digital content,
wherein
the genome information includes host genome information, microbiome, and host epigenome information, and
the customized digital content, which is visual content used to induce a psychological or mental state of the specific individual to a normal state or a relaxed state, includes at least one of a background color, an object color, a character font, and an object design which are output to a personal terminal.

2. The method of claim 1, wherein the clinical information includes survey information input by the specific individual, medical information of the specific individual, and medical information of the specific individual's family.

3. The method of claim 2, wherein the survey information includes data on at least a plurality of items among stress resilience, inflammatory response, nervous system function, boost energy-level, blood sugar response to a meal, body mass composition, sleep, nutrient absorption, physical fatigue, and food allergy.

4. The method of claim 1, wherein the psychological type is one type according to the Myers-Briggs Type Indicator (MBTI).

5. The method of claim 1, wherein the content database is a color therapy database that stores color information corresponding to the mental health state and the psychological type.

6. The method of claim 1, wherein the analysis apparatus selects genes associated with a psychological type of an individual among the genome information and pre-processes the genes as input data for the first learning model, and the input data is input to the first learning model to determine the psychological type of the specific individual.

7. An apparatus for analyzing customized content using user multi-feature information, the apparatus comprising:
an input device configured to receive genome information, clinical information, and lifestyle habit information for a specific individual;
a storage device configured to store a first learning model that receives the genome information and outputs a psychological type of an analysis target and a second learning model that receives the genome information, the clinical information, and the lifestyle habit information and outputs a mental health state of the analysis target; and
a computing device configured to determine a psychological type of the specific individual by inputting the genome information of the specific individual to the first learning model, configured to determine the mental health state of the specific individual by inputting the genome information, the clinical information, and the lifestyle habit information of the specific individual to the second learning model, and configured to determine customized digital content for the specific individual matching the psychological type and the mental health state with reference to a content database,
wherein
the genome information includes host genome information, microbiome information, and host epigenome information, and
the second learning model outputs the mental health state including at least one of apathy, depression, stress, chronic fatigue, and anxiety.

8. The apparatus of claim 7, wherein the customized digital content is visual content that induces a psychological or mental state of the specific individual to a normal state or a relaxed state and includes at least one of a background color, an object color, a character font, and an object design output to a personal terminal.

9. The apparatus of claim 7, wherein the clinical information includes survey information input by the specific individual, medical information of the specific individual, and medical information of the specific individual's family.

10. The apparatus of claim 9, wherein the survey information includes data on at least a plurality of items among stress resilience, inflammatory response, nervous system function, boost energy-level, blood sugar response to a meal, body mass composition, sleep, nutrient absorption, physical fatigue, and food allergy.

11. The apparatus of claim 7, wherein the content database is a color therapy database that stores color information corresponding to the mental health state and the psychological type.

12. The apparatus of claim 7, wherein the computing device selects genes associated with a psychological type of an individual among the genome information and pre-processes the genes as input data for the first learning model, and the input data is input to the first learning model.
